# EUROPÄISCHE PATENTSCHRIFT

(11) **EP 0 479 730 B1**
(45) Veröffentlichungstag und Bekanntmachung des Hinweises auf die Patenterteilung: **19.04.1995**
(21) Anmeldenummer: 91810774.9
(22) Anmeldetag: 01.10.1991
(51) Int. Cl.: A61M 29/02

(54) **Ballondilationskatheter**
Balloon dilatation catheter
Cathéter dilatateur à ballonnet

(30) Priorität: 04.10.1990 CH 3204/90
(43) Veröffentlichungstag der Anmeldung: 08.04.1992
(73) Patentinhaber: SCHNEIDER (EUROPE) AG, 8180 Bülach (CH)
(72) Erfinder: Goy, Jean-Jacques, CH-1800 Vevey (CH)
(74) Vertreter: Groner, Manfred

(56) Entgegenhaltungen:
- EP-A- 0 244 955
- EP-A- 0 277 370
- US-A- 4 909 258
- US-A- 4 944 745

## Beschreibung

Die Erfindung betrifft einen Ballondilatationskatheter für die perkutane transluminale Angioplastie nach dem Oberbegriff des unabhängigen Patentanspruchs 1.

Ein Katheter dieser Gattung ist im stand der Technik durch die EP-A-0 347 023 bekannt. Dieser wiest am distalen Ende eines einlumigen Schaftes einen Dilatationsballon auf. Ein Stützdraht ist fest mit dem Katheter verbunden.

Katheter dieser Gattung sind unter der Bezeichnung "PTCA-Katheter" zur Behandlung von Verengungen in Blutgefässen seit langem bekannt und haben sich im allgemeinen bewährt. Die Behandlung von Verengungen oder Stenosen in Verzweigungen sind jedoch mit den bekannten Kathetern sehr schwierig und komplex. Bekannt geworden sind insbesondere die beiden nachfolgenden Verfahren.

Bei der sogenannten "Kissing Balloon"-Technik werden zwei Ballondilatationskatheter gleichzeitig perkutan eingeführt.

Vor der Verzweigung trennen sich die distalen Enden der Dilatationskatheter, so dass in jeden Ast der Verzweigung ein Ballon zuliegen kommt. Die proximalen Enden der beiden Ballone berühren sich hierbei. In der Regel werden zur Dilatation der Stenose beide Ballondilatationskatheter gleichzeitig dilatiert. Dieses Verfahren und ein dazu vorgesehener Katheter ist in der oben ernähnten EP-A 0 347 023 beschrieben.

Nach der anderen Behandlungsmethode werden ebenfalls perkutan zwei gleiche Führungsdrähte eingeführt, und zwar so, dass sich diese vor der Verzweigung trennen. Auf dem einen Führungsdraht wird nun ein sogenannter "Monorail"-Katheter zuerst in den einen Ast der Verzweigung eingeführt. Die Verengungsstelle dieses Astes wird mit dem Ballon des Katheters dilatiert und nachher wird der Katheter vollständig zurückgezogen. Auf dem anderen Führungsdraht wird nun ein weiterer Ballondilatationskatheter dieser Art in den anderen Ast der Verzweigung eingeführt und nun die Engstelle dieses anderen Astes dilatiert. Ein Vorteil dieser Methode wird darin gesehen, dass die für die Fluoroskopie notwendige Zeit verkürzt werden kann. Die Behandlungszeit kann bei dieser Methode deshalb besonders kurz gehalten werden.

Der Erfindung liegt die Aufgabe zugrunde, einen Ballondilatationskatheter der genannten Gattung zu schaffen, der eine noch einfachere und dennoch sichere Behandlung von Engstellen in Arterienverzweigungen erlaubt und der trotzdem einfach und kostengünstig hergestellt werden kann. Die Aufgabe wird durch die Erfindung gemäss Anspruch 1 gelöst. Der erfindungsgemässe Ballondilatationskatheter weist somit im Schaft ein weiteres vor dem proximalen Ende des Ballons nach aussen mündendes Lumen auf. Dieses Lumen ist am proximalen Ende des Schaftes mit einem Anschlussstück verbunden, durch welches in dieses weitere Lumen ein Führungsdraht einführbar ist. Zur Behandlung einer Stenose an einer Arterienverzweigung wird der erfindungsgemässe Ballondilatationskatheter in einen der Aeste der Verzweigung und der Führungsdraht durch das weitere Lumen in den anderen Ast eingeführt. Die Engstelle im ersten Ast wird wie üblich durch Dehnung des Ballons dilatiert. Ist eine weitere Dilatation nicht notwendig, so wird der Katheter wie üblich vollständig zurückgezogen. Ist jedoch auch eine Dilatation im anderen Ast der Verzweigung erforderlich, so wird der Katheter zurückgezogen und ein weiterer, üblicher Dilatationskatheter auf dem bereits eingesetzten Führungsdraht in den zu dilatierenden weiteren Ast eingeführt. Wesentlich ist nun, dass der Führungsdraht gewährleistet, dass auch nach der Dilatation des ersten Astes in den möglicherweise weiter verengten zweiten Ast ein Katheter einführbar ist. Zudem kann durch das genannte weitere Lumen mit oder ohne eingeführtem Führungsdraht ein Medikament oder ein Kontrastmittel eingeführt werden. Ebenfalls ist eine Blutdruckmessung oder eine andere Messung über dieses weitere Lumen möglich.

Der erfindungsgemässe Ballondilatationskatheter weist einen fest mit dem Ballon oder dem Schaft verbundenen Stützdraht auf. Ballondilatationskatheter mit einem Stützdraht sind an sich bekannt und besitzen den Vorteil, dass sie mit sehr kleinem Profil hergestellt werden können, da im Ballon kein Schaft erforderlich ist. Nachteilig ist bei diesen Kathetern jedoch, dass eine Einführung von Kontrastmitteln oder Medikamenten oder eine Blutdruckmessung nicht möglich ist. Bei der erfindungsgemässen Ausführung kann jedoch jederzeit durch das weitere Lumen ein Behandlungsmittel in das Gefäss eingeführt oder eine Messung durchgeführt werden. Der erfindungsgemässe Ballondilatationskatheter kann somit auch zur Behandlung von Engstellen, die nicht in Gefässverzweigungen angeordnet sind, vorteilhaft sein. In solchen Fällen kann, falls gewünscht, die Steifigkeit des Katheters durch die Einführung eines Führungsdrahtes in das zusätzliche Lumen verstärkt werden. Das gleichzeitige Vorhandensein des Stützdrahtes und des Führungsdrahtes verleiht dem Katheter dann eine besonders hohe Steifigkeit.

Nach einer Weiterbildung der Erfindung ist die Öffnung, mit der das weitere Lumen nach außen mündet unmittelbar vor dem proximalen Ende des Ballons angeordnet. Das weitere Lumen erstreckt sich somit bei dieser Ausführung im wesentlichen über die ganze Länge des Schaftes. Ein in dieses Lumen eingeführter Führungsdraht versteift entsprechend den Schaft im wesentlichen auf seiner gesamten Länge.

Die Erfindung betrifft ebenfalls eine Anordnung mit einem Katheter nach Anspruch 1 und einem austauschbaren Führungsdraht. Diese Anordnung ist dadurch gekennzeichnet, dass am proximalen Ende des Schaftes ein erstes Anschlussstück mit einer Abzweigung für den Anschluss einer Pumpe zur Dilatation bzw. Faltung des Ballons und eine Abzweigung für ein zweites Anschlussstück angeordnet ist. Das zweite Anschlussstück besitzt eine Abzweigung für den Anschluss eines Messgerätes oder eines Gerätes für die Zuführung eines Kontrastmittels oder Medikamentes in das weitere Lumen des Schaftes und einen Eingang zur Einführung des Führungsdrahtes ebenfalls in das weitere Lumen. Eine solche Anordnung erlaubt unabhängig voneinander die Durchführung von Messungen oder Infusionen über das weitere Lumen und die Regulierung des Druckes im Ballon über das erste Lumen. Unabhängig vom Druck im Ballon kann über das weitere Lumen der Führungsdraht eingeführt werden. Diese Funktionen sind auch dann möglich, wenn der Katheter einen festen Stützdraht aufweist.

Weitere vorteilhafte Merkmale ergeben sich aus der nachfolgenden Beschreibung. Ein Ausführungsbeispiel der Erfindung wird nachfolgend anhand der Zeichnungen näher erläutert. Es zeigen:
- Fig. 1: teilweise im Längsschnitt eine erfindungsgemässe Katheteranordnung,
- Fig. 2: ein Schnitt entlang der Linie II-II in Fig. 1,
- Fig. 3: ein Schnitt entlang der Linie III-III in Fig. 1,
- Fig. 4: schematisch eine stenosierte Arterienverzweigung mit eingeführtem Katheter gemäss der Erfindung, und
- Fig. 5 und 6: die Arterienverzweigung gemäss Fig. 4, jedoch während der Behandlung des anderen Verzweigungsastes.

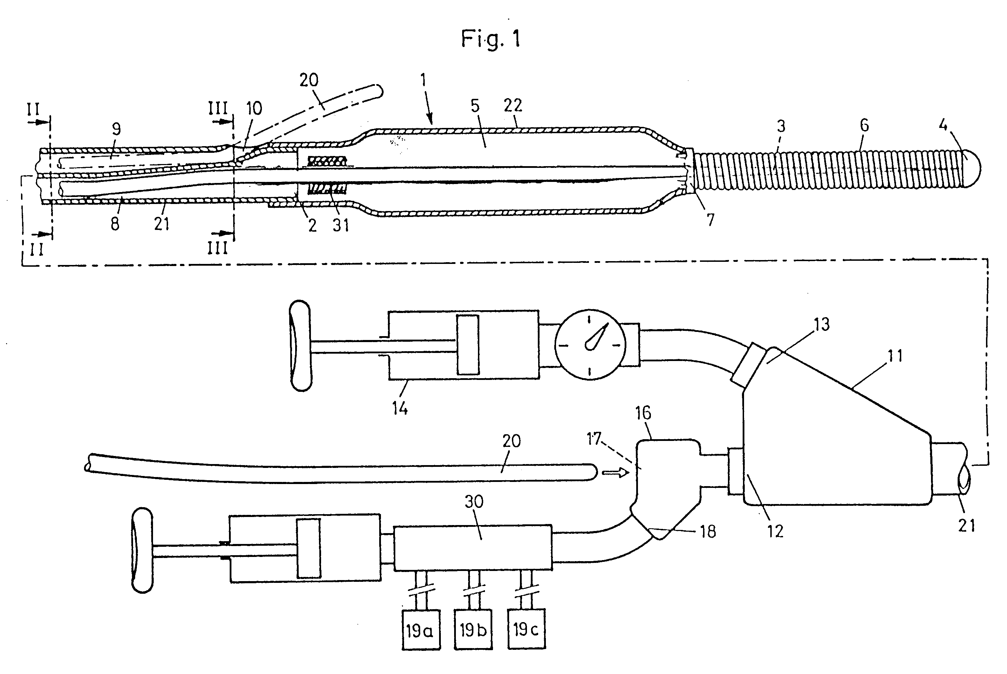

Die in Fig. 1 gezeigte Katheteranordnung besteht aus einem Ballondilatationskatheter 1, einem austauschbaren Führungsdraht 20, einer Pumpe 14 und einem Gerät 19 für eine Blutdruckmessung oder zur Einführung von Kontrastmittel oder Medikamenten.

Der Ballondilatationskatheter 1 weist an seinem distalen Ende einen Ballon 22 auf, der an seinem proximalen Ende auf einen Schaft 21 aufgeschoben und mit diesem thermoplastisch verschweisst oder verklebt ist. Der Ballon 22 besteht beispielsweise aus einem geeigneten Polyamid, aus Polyaethylenterephthalat oder aus einem anderen geeigneten Polymer. Er weist vorzugsweise ein schmales Profil auf. Zu seiner Dilatation wird der Innenraum 5 des Ballons 22 unter einem Druck von beispielsweise 8 bar mit einer Flüssigkeit gefüllt. Dazu ist der Innenraum 5 über eine Oeffnung 2 und ein erstes Lumen 8 mit der Pumpe 14 verbunden.

Am distalen Ende ist der Ballon 22 an der Stelle 7 fest mit einer flexiblen Spiralfeder 6 verbunden. Im Innern dieser Feder 6 verläuft das distale Ende eines Stützdrahtes 3, der an seiner Spitze an der Stelle 4 mit der Spiralfeder 6 verlötet oder verschweisst ist. Im Bereich des Ballons 22 und der Spiralfeder 6 ist der Stützdraht 3 konisch verjüngt. Der Stützdraht 3 erstreckt sich im ersten Lumen 8 vorzugsweise über die ganze Länge des Schaftes 21 und ist an seinem proximalen Ende fest mit dem Schaft 21 verbunden. Der an seiner Spitze biegbare Stützdraht 3 sorgt für die Steuerbarkeit und Drehmomentübertragung vom proximalen zum distalen Ende des Katheters 1 bei seiner Einführung. Zusammen mit der Spiralfeder 6 erlaubt er die Einführung des Ballons 22 in Gefässbiegungen oder in einen Ast einer Verzweigung. Geeignete Markierungen 31 am proximalen Ende des Ballondilatationskatheters 1 erlauben in bekannter Weise die Beobachtung der Position des Ballons 22 mit einer Röntgenanlage.

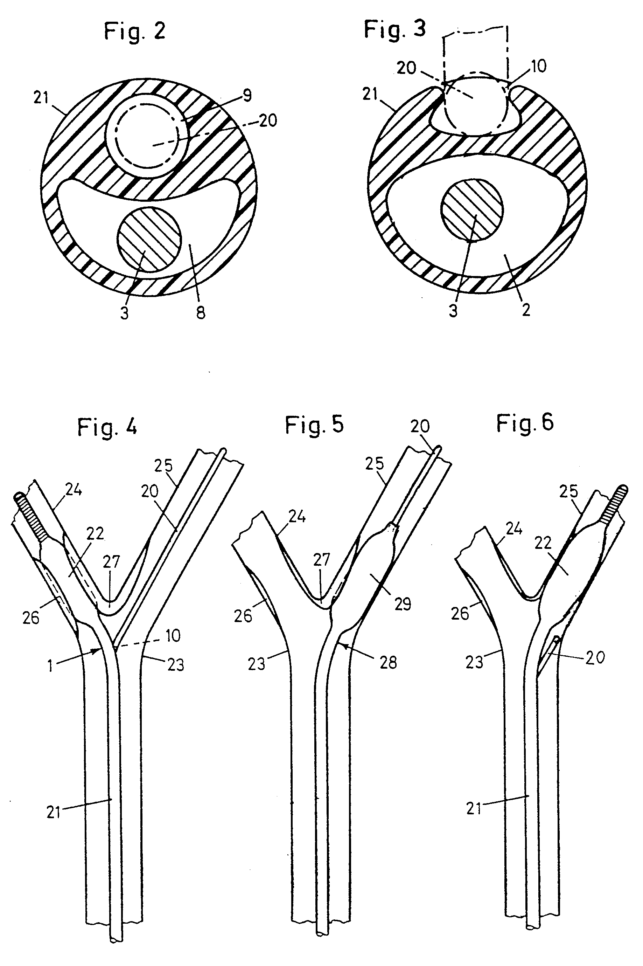

Der Schaft 21 weist ein zweites Lumen 9 auf, das sich über die ganze Länge des Schaftes erstreckt und das gemäss Fig. 2 vorzugsweise einen runden Querschnitt aufweist. Das Lumen 9 mündet unmittelbar hinter dem proximalen Ende des Ballons 22 mit einer Oeffnung 10 nach aussen. Das Lumen 9 und das Balloninnere 5 sind somit voneinander getrennt. An seinem proximalen Ende ist das zweite Lumen 9 mit einem Anschlussstück 16 verbunden. Dieses Anschlussstück 16 besitzt eine flüssigkeitsdichte Hahnbank 30, mit der das Lumen 9 über eine Abzweigung 18 wahlweise zur Blutdruckmessung, zur Injektion eines Kontrastmittels oder eines Medikamentes mit einem der Geräte 19 verbindbar ist. Durch die Oeffnung 17, in der eine Dichtungsmembran angeordnet ist, kann der Führungsdraht 20 in das Lumen 9 eingeschoben werden.

Der Schaft 21 kann ebenfalls aus einem geeigneten Polyamid, aus Polyaethylen oder einem anderen geeigneten thermoplastischen Polymer hergestellt sein. Sein Aussendurchmesser beträgt beispielsweise 1,2 mm und seine Länge beispielsweise 130 cm. Der Innendurchmesser des kreisförmigen Lumens 9 beträgt vorzugsweise etwa 0,4 mm und der Aussendurchmesser des Führungsdrahtes 20 vorzugsweise etwa 0,35 mm. Die engste Weite des ersten Lumens 8 beträgt vorzugsweise etwa 0,5 mm. Der Aussendurchmesser des Stützdrahtes 3 ist im Bereich des Schaftes 21 entsprechend etwas kleiner. Wie die Fig. 2 und 3 zeigen, ist der Querschnitt des ersten Lumens 8 halbkreisförmig oder halbmondförmig, so dass auch bei kleinem Aussendurchmesser des Schaftes 21 im Lumen 8 Druckflüssigkeit für den Ballon 22 zirkulieren kann. Der Schaft 21 ist wie gezeigt vorzugsweise zweilumig, es sind jedoch Ausführungen denkbar, bei denen der Schaft 21 mehr als zwei Lumen aufweist.

An seinem proximalen Ende ist der Schaft 21 mit einem ersten Anschlussstück 11 mit dem zweiten Anschlussstück 16 verbunden. Das erste Anschlussstück 11 besitzt in bekannter Weise zwei Abzweigungen 12 und 13, wobei die Abzweigung 12 die Verbindung des Lumens 9 mit dem Anschlussstück 16 und die Abzweigung 13 die Verbindung des Lumens 8 mit der Pumpe 14 gewährleisten. Solche Anschlussstücke 11 sind an sich gut bekannt.

Eine bevorzugte Verwendung des Ballondilatationskatheters und der Katheteranordnung wird nachfolgend anhand der Fig. 4 und 5 näher erläutert.

Eine Arterienverzweigung 23 weist einen linken Ast 24 und einen rechten Ast 25 auf. Die Verzeigung ist durch stenosierte Bereiche 26 und 27 verengt. Um die Verzweigungsstelle wieder durchgängig zu machen, werden beide Aeste 24 und 25 dilatiert. Mit der erfindungsgemässen Katheteranordnung sind nun die folgenden Verfahren möglich.

Nach einem ersten Verfahren wird der Ballon 22 des Katheters 1 beispielsweise in den Ast 24 eingeführt. Da wie oben erwähnt der Katheter 1 einen Stützdraht 3 aufweist, der im wesentlichen ein fester Führungsdraht 3 ist, kann der Katheter 1 gesteuert und der Ballon 22 in den Ast 24 eingeführt werden. Anschliessend wird nun der Führungsdraht 20 in den Katheter eingeführt und so vorgeschoben, dass er wie in Fig. 4 gezeigt durch die Verengung 27 in den Ast 25 ragt. Das distale Ende des Führungsdrahtes 20 ragt somit durch die Oeffnung 10 des Schaftes 21 nach aussen und in den Ast 25. Der Führungsdraht 20 ist ein steuerbarer an sich bekannter Führungsdraht. Ist der Ballon 22 in der geeigneten Position, so kann in bekannter Weise durch Dilatation des Ballons 22 der verengende Bereich 26 radial nach aussen verdrängt werden. Hierbei wird in der Regel der Ast 25 weiter verengt. Der liegende Draht 20 verhindert jedoch eine vollständige Verengung und gewährleistet, dass der Ast 25 für die Einführung eines weiteren Katheters offen bleibt. Nach dem Dilatieren des Astes 24 wird der Katheter 1 vollständig zurückgezogen, so dass lediglich noch der Führungsdraht 20 eingesetzt ist. Anschliessend wird nun auf dem Führungsdraht 20 ein üblicher Ballondilatationskatheter 28 eingeführt und der Ballon 29 so in den Ast 25 eingeschoben, dass die Engstelle 27 dilatiert werden kann. Die Einführung des Katheters 28 ist einfach, da er durch den bereits eingesetzten Führungsdraht 20 geführt ist. Nach dem Dilatieren der Engstelle 27 wird der Katheter 28 zusammen mit dem Führungsdraht 20 entfernt. Es ist auch möglich, nach dem entfernen des Katheters 28 nochmals einen Katheter 1 in den Ast 24 einzuführen, um diesen nochmals zu dilatieren.

Nach dem zweiten Verfahren wird zuerst ein Führungsdraht 20 in einen der Aeste 24 oder 25 und auf diesem ein Ballon 29 eines üblichen Katheters 28 in den entsprechenden Ast eingeführt. Nach diesem Verfahren wird somit zuerst einer der Aeste 24 oder 25 mit dem üblichen Ballondilatationskatheter 28 dilatiert, wie dies in Fig. 5 schematisch dargestellt ist. Anschliessend wird der Katheter 28 vollständig zurückgezogen, wobei der Führungsdraht 20 im Gefäss verbleibt. Anschliessend wird der erfindungsgemässe Katheter 1 auf dem Führungsdraht 20 so eingeführt, dass sich sein Ballon 22 wie in Fig. 4 gezeigt im anderen Ast befindet. Ist der Ballon 22 positioniert, so wird mit diesem wie oben beschrieben dieser andere Ast dilatiert. Anschliessend wird der Katheter 1 und der Führungsdraht 20 vollständig zurückgezogen.

Nach einem dritten Verfahren wird wie in Fig. 4 gezeigt, der eine Ast 24 dilatiert. Zur Behandlung des anderen Astes 25 wird nun der Ballonkatheter 1 nur soweit zurückgezogen, dass sein Ballon 22 in diesen anderen Ast 25 eingeführt werden kann. Der Ballon 22 liegt dann neben dem vorderen Ende des Führungsdrahtes 20 im Ast 25, wie in Fig. 6 gezeigt. Durch Entfaltung des Ballons 22 wird nun der Ast 25 dilatiert, wobei der Führungsdraht 20 vorher aus dem Ast 25 zurückgezogen wird.

Der erfindungsgemässe Katheter ist auch bei der Behandlung von Stenosen in nicht verzweigten Gefässabschnitten vorteilhaft. Durch sein vergleichsweise kleines Profil lässt sich der gefaltete Ballon leichter in enge Stenosen und Gefässabschnitte einführen. Gegenüber den bekannten Kathetern mit festem Stützdraht erlaubt er zudem einen wesentlich einfacheren Wechsel des Ballonkatheters, wenn beispielsweise ein grösserer Ballon erforderlich ist. In diesem Fall kann der Führungsdraht 20 beim Katheterwechsel im Gefäss verbleiben. Beim Zurückziehen des nicht mehr benötigten Ballonkatheters bleibt der Führungsdraht 20 im Gefäss. Der neue Ballonkatheter kann dann schonend und vergleichsweise schnell auf diesem Führungsdraht zur Engstelle vorgeschoben und in diese eingeführt werden.

## Patentansprüche

1. Ballondilatationskatheter mit einem Schaft (21), mit einem durch den Schaft (21) sich erstreckenden ersten Lumen (8) zum Dilatieren eines am distalen Ende des Schaftes (21) angebrachten Dilatationsballons (22), und mit einem fest mit dem Ballon (22) und/oder dem Schaft (21) verbundenen Stützdraht (3), dadurch gekennzeichnet, dass der Schaft (21) ein weiteres, vor dem proximalen Ende des Dilatationsballons (22) mit einer Oeffnung (10) nach aussen mündendes Lumen (9) aufweist, das am proximalen Ende des Schaftes (21) mit einem Anschlussstück (16) verbunden ist, durch welches durch dieses weitere Lumen (9) ein Führungsdraht (20) hindurchführbar ist.

2. Katheter nach Anspruch 1, dadurch gekennzeichnet, dass die Oeffnung (10) unmittelbar vor dem proximalen Ende des Dilatationsballons (22) angeordnet ist.

3. Katheter nach Anspruch 1 oder 2, dadurch gekennzeichnet, dass das erste Lumen (8) im Querschnitt etwa halbkreisförmig oder halbmondförmig und das zweite Lumen (9) im Querschnitt etwa kreisförmig ist.

4. Anordnung mit einem Katheter nach Anspruch 1 und einem austauschbaren steuerbaren Führungsdraht, dadurch gekennzeichnet, dass am proximalen Ende des Schaftes (21) ein erstes Anschlussstück (11) mit einer Abzweigung (13) für den Anschluss einer Druck/Saugpumpe (14) zur Dilatation bzw. Faltung des Dilatationsballons (22) und mit einer Abzweigung (12) für ein zweites Anschlussstück (16) vorgesehen ist und dass das zweite Anschlusstück (16) eine Abzweigung (18) für den Anschluss eines Messgerätes (19) oder eines Gerätes für die Zuführung eines Kontrastmittels oder Medikamentes in das weitere Lumen (9) sowie einen flüssigkeitsdicht verschliessbaren Eingang (17) zur Einführung des Führungsdrahtes (20) in das weitere Lumen (9) aufweist.

## Claims

1. Balloon dilatation catheter having a shank (21), a first lumen (8) extending through the shank (21) for dilating a dilatation balloon (22) mounted at the distal end of the shank (21), and a support wire (3) fixedly connected to the balloon (26) and/or the shank (21), characterised in that the shank (21) has another lumen (9) having an opening (10) which opens outwardly before the proximal end of the dilatation balloon (22), said lumen (9) being connected at the proximal end of the shank (21) to a connector (16) through which a guide wire (20) can be passed through said additional lumen (9).

2. Catheter according to claim 1, characterised in that the opening (10) is provided immediately in front of the proximal end of the dilatation balloon (22).

3. Catheter according to claim 1 or 2, characterised in that the first lumen (8) is substantially semicircular or semilunar in cross section and the second lumen (9) is substantially circular in cross section.

4. Arrangement with a catheter according to claim 1 and an exchangeable, controllable guide wire, characterised in that, at the proximal end of the shank (21), a first connector (11) is provided, having a branch (13) for the attachment of a pressure/suction pump (14) for dilating or collapsing the dilatation balloon (22) and having a branch (12) for a second connector (16), and in that the second connector (16) has a branch (18) for the attachment of a measuring device (19) or a device for introducing a contrast agent or drug into the additional lumen (9) and an inlet (17), capable of being sealed in fluidtight manner, for inserting the guide wire (20) into the additional lumen (9).

## Revendications

1. Cathéter dilatateur à ballonnet comportant une tige (21) avec une première lumière (8) s'étendant à travers la tige (21) pour la dilatation d'un ballonnet de dilatation (22) appliqué sur l'extrémité distale de la tige (21), et avec un fil de support (3) raccordé solidement au ballonnet (22) et/ou à la tige (21), caractérisé en ce que la tige (21) présente une autre lumière (9) débouchant vers l'extérieur, avant l'extrémité proximale du ballonnet de dilatation (22) avec une ouverture (10), lumière qui sur l'extrémité proximale de la tige (21) est reliée à un élément de raccordement (16) à travers lequel un fil de guidage (20) pourra être introduit par cette autre lumière (9).

2. Cathéter selon la revendication 1, caractérisé en ce que l'ouverture (10) est disposée directement en amont de l'extrémité proximale du ballonnet de dilatation (22).

3. Cathéter selon la revendication 1 ou 2, caractérisé en ce que la première lumière (8) dans sa section transversale est de forme sensiblement semi-circulaire ou en forme de demi-lune et la deuxième lumière (9) est en section transversale de forme sensiblement circulaire.

4. Dispositif comprenant un cathéter selon la revendication 1 et un fil de guidage remplaçable ou échangeable et contrôlable, caractérisé en ce que sur l'extrémité proximale de la tige (21) il est prévu un premier élément de raccordement (11) avec une dérivation (13) pour le raccordement d'une pompe pression/aspiration (14) pour la dilatation ou le pliage du ballonnet de dilatation (22) et avec une dérivation (12) pour un deuxième tronçon de raccordement (16) et en ce que le deuxième tronçon de raccordement (16) comporte une dérivation (18) pour le raccordement d'un appareil de mesure (19) ou d'un appareil pour l'amenée d'un agent de contraste ou d'un médicament dans l'autre lumière (9) ainsi qu'une entrée (17) obturable étanche au liquide pour l'introduction du fil de guidage (20) dans l'autre lumière (9).
